# EUROPEAN PATENT SPECIFICATION

(11) **EP 3 242 652 B1**
(45) Date of publication and mention of the grant of the patent: **02.08.2023**
(21) Application number: 16734158.5
(22) Date of filing: 24.06.2016
(51) Int. Cl.: A61K 8/27, A61K 8/44, A61Q 11/00, A61K 8/21

(54) **ORAL CARE COMPOSITIONS AND METHODS OF USE**
MUNDPFLEGEZUSAMMENSETZUNGEN UND VERFAHREN ZUR VERWENDUNG
COMPOSITIONS DE SOINS BUCCO-DENTAIRES ET LEURS PROCÉDÉS D'UTILISATION

(30) Priority: 01.07.2015 US 201562187801 P
(43) Date of publication of application: 15.11.2017
(73) Proprietor: Colgate-Palmolive Company, New York, NY 10022 (US)
(72) Inventor: PRENCIPE, Michael, Princeton Junction, New Jersey 08550 (US); RUSSO, Amy, Belle Mead, New Jersey 08502 (US); STETTLER, Hansruedi, 4054 Basel (CH); MORGAN, Andre Michelle, Robbinsville, New Jersey 08690 (US)
(74) Representative: Wibbelmann, Jobst
(86) International application number: PCT/US2016/039194
(87) International publication number: WO 2017/003844

(56) References cited:
- WO-A1-2014/088572
- WO-A1-2014/088575
- WO-A1-2014/098822
- WO-A1-2015/094849
- WO-A1-2016/105440

## Description

### FIELD

This invention relates to oral care compositions comprising arginine, zinc oxide and zinc citrate, a fluoride source, and abrasive silica as defined in the claims.

### BACKGROUND

Oral care compositions present particular challenges in preventing microbial contamination. Arginine and other basic amino acids have been proposed for use in oral care and are believed to have significant benefits in combating cavity formation and tooth sensitivity.

Commercially available arginine-based toothpaste contains arginine bicarbonate and precipitated calcium carbonate, but not fluoride. The carbonate ion is believed to have cariostatic properties, and the calcium is believed to form a complex with arginine to provide a protective effect.

However, the formulation of certain oral care compositions presents special challenges. For example, oral care compositions comprising arginine or basic amino acids may have a basic pH, increasing potential for microbial contamination compared to acidic formulations. Moreover, not all preservatives are active at higher pH. Some preservatives negatively affect the taste or aesthetics of the product. While certain preservatives, such as ethanol or parabens, are known to be effective at a range of pHs, these preservatives are not suitable for all products or all markets.

Zinc is a well-known antimicrobial agent used in toothpaste compositions Zinc is also a well-known essential mineral for human health, and has been reported to help strengthen dental enamel and to promote cell repair. Unfortunately, conventional toothpaste formulations often require a high concentrations of zinc, e.g., 2% by weight or more, to achieve efficacy. At this concentration, the zinc imparts a notably astringent taste to the composition. There is thus a need for improved antibacterial toothpaste formulations that do not suffer from the drawbacks of conventional compositions.

Accordingly, there is a need for improved preservative agents for use in oral compositions comprising basic amino acids.

### BRIEF SUMMARY

It has been surprisingly found that the inclusion of arginine unexpectedly increases the antibacterial effect of oral care compositions comprising zinc oxide and zinc citrate, selected at certain amounts as defined in the claims in the oral cavity of a user. The formulations use comparable amounts of zinc to what is found in current market formulations. However, while comparable amounts of zinc are used in the current invention (i.e., relative to various market formulations), the amount of soluble zinc is believed to be actually increased relative to various market formulations. Without being bound by any theory, it is believed that the presence of arginine may help to increase the amount of available soluble zinc, which aids in delivery and inhibits bacterial growth in the oral cavity of a user.

The present invention refers to an oral care composition (Composition 1.0) comprising:
a. about 4 wt% arginine, based on the total weight of the composition, the weight of the basic amino acid being calculated as free form,
b. zinc oxide and zinc citrate, wherein the weight ratio of the amount of zinc oxide to zinc citrate is 2: 1,
c. a fluoride source, wherein the fluoride source is sodium fluoride or stannous fluoride from 0.1 wt% - 2 wt%, and
d. an abrasive silica.

A composition for use as set for in any of the preceding compositions.

In another embodiment, the invention encompasses the oral care composition of the invention for use in a method to improve oral health comprising applying an effective amount of the oral composition of any of the embodiments set forth above to the oral cavity of a subject in need thereof, i.e., in a method to
i. reduce or inhibit formation of dental caries,
ii. reduce, repair or inhibit early enamel lesions, e.g., as detected by quantitative light-induced fluorescence (QLF) or electrical caries measurement(ECM),
iii. reduce or inhibit demineralization and promote remineralization of the teeth,
iv. reduce hypersensitivity of the teeth,
v. reduce or inhibit gingivitis,
vi. promote healing of sores or cuts in the mouth,
vii. reduce levels of acid producing bacteria,
viii. to increase relative levels of arginolytic bacteria,
ix. inhibit microbial bio film formation in the oral cavity,
x. raise and/or maintain plaque pH at levels of at least pH 5.5 following sugar challenge,
xi. reduce plaque accumulation,
xii. treat dry mouth,
xiii. enhance systemic health, including cardiovascular health,e.g., by reducing potential for systemic infection via the oral tissues,
xiv. Whiten teeth,
xv. reduce erosion of the teeth,
xvi. immunize (or protect) the teeth against cariogenic bacteria and their effects, and/or
xvii. clean the teeth and oral cavity.

### DETAILED DESCRIPTION

As used herein, the term "oral composition" means the total composition that is delivered to the oral surfaces. The composition is further defined as a product which, during the normal course of usage, is not, the purpose of systemic administration of particular therapeutic agents, intentionally swallowed, but is rather retained in the oral cavity for a time sufficient to contact substantially all of the dental surfaces and/or oral tissues for the purposes of oral activity. Examples of such compositions include, but are not limited to, toothpaste or a dentifrice, a mouthwash or a mouth rinse, a topical oral gel, a denture cleanser, and the like.

As used herein, the term "dentifrice" means paste, gel, or liquid formulations unless otherwise specified. The dentifrice composition can be in any desired form such as deep striped, surface striped, multi-layered, having the gel surrounding the paste, or any combination thereof. Alternatively the oral composition is provided as a dual phase composition, wherein individual compositions are combined when dispensed from a separated compartment dispenser.

### Basic Amino Acids

The basic amino acid used according to the invention is arginine.

In certain embodiments, the arginine is L-arginine, or a salt thereof.

The compositions of the invention are intended for topical use in the mouth and so salts for use in the present invention should be safe for such use, in the amounts and concentrations provided. Suitable salts include salts known in the art to be pharmaceutically acceptable salts which are generally considered to be physiologically acceptable in the amounts and concentrations provided. Physiologically acceptable salts include those derived from pharmaceutically acceptable inorganic or organic acids or bases, for example acid addition salts formed by acids which form a physiological acceptable anion, e.g., hydrochloride or bromide salt, and base addition salts formed by bases which form a physiologically acceptable cation, for example those derived from alkali metals such as potassium and sodium or alkaline earth metals such as calcium and magnesium. Physiologically acceptable salts may be obtained using standard procedures known in the art, for example, by reacting a sufficiently basic compound such as an amine with a suitable acid affording a physiologically acceptable anion.

### Fluoride Ion Source

The oral care compositions include a fluoride source, wherein the fluoride source is sodium fluoride or stannous fluoride from 0.1 wt% - 2 wt%. General examples of fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al.

### Surfactants

The invention may in some embodiments contain anionic surfactants, e.g., the Compositions of Composition 1.0, *et seq*., for example, water-soluble salts of higher fatty acid monoglyceride monosulfates, such as the sodium salt of the monosulfated monoglyceride of hydrogenated coconut oil fatty acids such as sodium N- methyl N-cocoyl taurate, sodium cocoglyceride sulfate; higher alkyl sulfates, such as sodium lauryl sulfate; higher alkyl-ether sulfates, e.g., of formula CH₃(CH₂)ₘCH₂(OCH₂CH₂)ₙOS0₃X, wherein m is 6-16, e.g., 10, n is 1-6, e.g., 2, 3 or 4, and X is Na or, for example sodium laureth-2 sulfate (CH₃(CH2)₁₀CH₂(OCH₂CH₂)₂OS0₃Na); higher alkyl aryl sulfonates such as sodium dodecyl benzene sulfonate (sodium lauryl benzene sulfonate); higher alkyl sulfoacetates, such as sodium lauryl sulfoacetate (dodecyl sodium sulfoacetate), higher fatty acid esters of 1,2 dihydroxy propane sulfonate, sulfocolaurate (N-2- ethyl laurate potassium sulfoacetamide) and sodium lauryl sarcosinate. By "higher alkyl" is meant, e.g., C₆-₃o alkyl. In particular embodiments, the anionic surfactant (where present) is selected from sodium lauryl sulfate and sodium ether lauryl sulfate. When present, the anionic surfactant is present in an amount which is effective, e.g., > 0.001% by weight of the formulation, but not at a concentration which would be irritating to the oral tissue, e.g., 1 %, and optimal concentrations depend on the particular formulation and the particular surfactant. In one embodiment, the anionic surfactant is present at from 0.03% to 5% by weight, e.g., 1.5%.

In another embodiment, cationic surfactants useful in the present invention can be broadly defined as derivatives of aliphatic quaternary ammonium compounds having one long alkyl chain containing 8 to 18 carbon atoms such as lauryl trimethylammonium chloride, cetyl pyridinium chloride, cetyl trimethylammonium bromide, diisobutylphenoxyethyldimethylbenzylammonium chloride, coconut alkyltrimethylammonium nitrite, cetyl pyridinium fluoride, and mixtures thereof. Illustrative cationic surfactants are the quaternary ammonium fluorides described in U.S. Pat. No. 3,535,421, to Briner et al.. Certain cationic surfactants can also act as germicides in the compositions.

Illustrative nonionic surfactants of Composition 1.0, *et seq.,* that can be used in the compositions of the invention can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials. In a particular embodiment, the composition of the invention comprises a nonionic surfactant selected from polaxamers (e.g., polaxamer 407), polysorbates (e.g., polysorbate 20), polyoxyl hydrogenated castor oils (e.g., polyoxyl 40 hydrogenated castor oil), and mixtures thereof.

Illustrative amphoteric surfactants of Composition 1.0, *et seq.,* that can be used in the compositions of the invention include betaines (such as cocamidopropylbetaine), derivatives of aliphatic secondary and tertiary amines in which the aliphatic radical can be a straight or branched chain and wherein one of the aliphatic substituents contains about 8-18 carbon atoms and one contains an anionic water-solubilizing group (such as carboxylate, sulfonate, sulfate, phosphate or phosphonate), and mixtures of such materials.

The surfactant or mixtures of compatible surfactants can be present in the compositions of the present invention in 0.1% to 5%, in another embodiment 0.3% to 3% and in another embodiment 0.5% to 2% by weight of the total composition.

### Flavoring Agents

The oral care compositions of the invention may also include a flavoring agent. Flavoring agents which are used in the practice of the present invention include, but are not limited to, essential oils and various flavoring aldehydes, esters, alcohols, and similar materials, as well as sweeteners such as sodium saccharin. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

The flavoring agent is incorporated in the oral composition at a concentration of 0.01 to 2% by weight.

### Chelating and anti-calculus agents

The oral care compositions of the invention also may include one or more chelating agents able to complex calcium found in the cell walls of the bacteria. Binding of this calcium weakens the bacterial cell wall and augments bacterial lysis.

Another group of agents suitable for use as chelating or anti-calculus agents in the present invention are the soluble pyrophosphates. The pyrophosphate salts used in the present compositions can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The salts are useful in both their hydrated and unhydrated forms. An effective amount of pyrophosphate salt useful in the present composition is generally enough to provide at least 0.1 wt. % pyrophosphate ions, e.g., 0.1 to 3 wt 5, e.g., 0.1 to 2 wt %, e.g., 0.1 to 1 wt%, e.g., 0.2 to 0.5 wt%. The pyrophosphates also contribute to preservation of the compositions by lowering the effect of water activity.

### Polymers

The oral care compositions of the invention also optionally include one or more polymers, such as polyethylene glycols, polyvinyl methyl ether maleic acid copolymers, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts. Certain embodiments include 1 :4 to 4: 1 copolymers of maleic anhydride or acid with another polymerizable ethylenically unsaturated monomer, for example, methyl vinyl ether (methoxyethylene) having a molecular weight (M.W.) of about 30,000 to about 1,000,000 (Mw). These copolymers are available for example as Gantrez AN 139(M.W. 500,000), AN 1 19 (M.W. 250,000) and S-97 Pharmaceutical Grade (M.W. 70,000), of GAF Chemicals Corporation.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1 103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone.

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorosorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid.

Another useful class of polymeric agents includes polyamino acids, particularly those containing proportions of anionic surface-active amino acids such as aspartic acid, glutamic acid and phosphoserine, as disclosed in U.S. Pat. No. 4,866,161 Sikes et al..

In preparing oral care compositions, it is sometimes necessary to add some thickening material to provide a desirable consistency or to stabilize or enhance the performance of the formulation. In certain embodiments, the thickening agents are carboxyvinyl polymers, carrageenan, xanthan gum, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Silica may also be available as a thickening agent, e.g., synthetic amorphous silica. Colloidal magnesium aluminum silicate or finely divided silica can be used as component of the thickening composition to further improve the composition's texture. In certain embodiments, thickening agents in an amount of about 0.5% to about 5.0% by weight of the total composition are used. Thickeners may be present in an amount of from 1 wt % to 15 wt %, from 3 wt % to 10 wt %, 4 wt % to 9 wt %, from 5 wt % to 8 wt %, for example 5 wt %, 6 wt %, 7 wt %, or 8 wt %.

### Abrasives

The oral care composition of the invention comprises abrasive silica. Natural calcium carbonate may additionally be used. Natural calcium carbonate is found in rocks such as chalk, limestone, marble and travertine. It is also the principle component of egg shells and the shells of mollusks. The natural calcium carbonate abrasive of the invention is typically a finely ground limestone which may optionally be refined or partially refined to remove impurities. For use in the present invention, the material has an average particle size of less than 10 microns, e.g., 3-7 microns, e.g. about 5.5 microns. For example a small particle silica may have an average particle size (D50) of 2.5 - 4.5 microns. Because natural calcium carbonate may contain a high proportion of relatively large particles of not carefully controlled, which may unacceptably increase the abrasivity, preferably no more than 0.01%, preferably no more than 0.004% by weight of particles would not pass through a 325 mesh. The material has strong crystal structure, and is thus much harder and more abrasive than precipitated calcium carbonate. The tap density for the natural calcium carbonate is for example between 1 and 1.5 g/cc, e.g., about 1.2 for example about 1.19 g/cc. There are different polymorphs of natural calcium carbonate, e.g., calcite, aragonite and vaterite, calcite being preferred for purposes of this invention. An example of a commercially available product suitable for use in the present invention includes Vicron ^{®} 25-11 FG from GMZ.

Precipitated calcium carbonate is generally made by calcining limestone, to make calcium oxide (lime), which can then be converted back to calcium carbonate by reaction with carbon dioxide in water. Precipitated calcium carbonate has a different crystal structure from natural calcium carbonate. It is generally more friable and more porous, thus having lower abrasivity and higher water absorption. For use in the present invention, the particles are small, e.g., having an average particle size of 1 - 5 microns, and e.g., no more than 0.1 %, preferably no more than 0.05% by weight of particles which would not pass through a 325 mesh. The particles may for example have a D50 of 3-6 microns, for example 3.8 - 4.9, e.g., about 4.3; a D50 of 1-4 microns, e.g. 2.2-2.6 microns, e.g., about 2.4 microns, and a D10 of 1-2 microns, e.g., 1.2-1.4, e.g. about 1.3 microns. The particles have relatively high water absorption, e.g., at least 25 g/l00g, e.g. 30-70 g/100g. Examples of commercially available products suitable for use in the present invention include, for example, Carbolag^{®} 15 Plus from Lagos Industria Quimica.

In certain embodiments the invention may comprise additional calcium-containing abrasives, for example calcium phosphate abrasive, e.g., tricalcium phosphate (Ca₃(PO₄)₂), hydroxyapatite (Ca₁₀(PO₄)₆(OH)₂), or dicalcium phosphate dihydrate (CaHPO₄ . 2H₂O, also sometimes referred to herein as DiCal) or calcium pyrophosphate, and/or silica abrasives, sodium metaphosphate, potassium metaphosphate, aluminum silicate, calcined alumina, bentonite or other siliceous materials, or combinations thereof.

The composition comprise an abrasive silica. Any silica suitable for oral care compositions may be used, such as small particle silica, precipitated silicas, or prophy silicas.

For example, the silica can also be small particle silica (e.g., Sorbosil AC43 from PQ, Warrington, United Kingdom). The composition preferable contains from 5 to 20 wt % small particle silica, or for example 10 - 15 wt %, or for example 5 wt %, 10 wt%, 15 wt % or 20 wt % small particle silica.

In another embodiment, the abrasive may be high cleaning precipitated silica having a pellicle cleaning ratio (PCR) of greater than 85 when tested at 20% loading is known in the art as high cleaning silica. Typically, high cleaning silica also has a mean particle size d₅₀ of from 5 to 15 µm and an oil absorption of from 40 to 120 cm³/100g silica. The cleaning efficacy of the precipitated silica is expressed using the pellicle cleaning ratio (PCR). This is typically measured at 20 % silica loading. The high cleaning silica preferably has a PCR value of greater than 85. The efficacy of the precipitated silica can also be expressed with reference to its abrasive characteristic using the radioactive dentin abrasion (RDA). Ideally, RDA values for an oral composition should be below about 250 to protect tooth enamel/dentin. Methods of performing PCR and RDA are described in *e.g.,* United States Patent Nos. 5,939,051 and 6,290,933 and "In Vitro Removal of Stain With Dentifrice", G. K. Stookey et al. , J. Dental Research, Vol. 61, pages 1236 - 9, November 1982." Typically, the precipitated silica has a mean particle size d₅₀ of from 5 to 15 µm and an oil absorption of from 40 to 120 cm³/100g silica. Examples of precipitated silica having a mean particle size d₅₀ of from 5 to 15 µm and an oil absorption of from 40 to 120 cm³/100g silica including commercially available silicas such as Zeodent^{®}103 and Zeodent^{®}105 (Huber Silica Americas).

The composition preferable contains from 5 to 20 wt % high cleaning precipitated silica, or for example 10 - 15 wt %, or for example 5 wt %, 10 wt%, 15 wt % or 20 wt % high cleaning precipitated silica.

The composition may also comprise an abrasive silica having an acid pH in the composition. For example, prophy silica available from Grace, offered as Sylodent^{™}, can be used. The acidic silica abrasive is included in the dentifrice components at a concentration of about 2 to about 35% by weight; about 3 to about 20 % by weight, about 3 to about 15% by weight, about 10 to about 15 % by weight. For example, the acidic silica abrasive may be present in an amount selected from 2 wt.%, 3wt.%, 4% wt.%, 5 wt.%, 6 wt.%, 7 wt.%, 8 wt.%, 9 wt.%, 10 wt.%, 11 wt.%, 12 wt.%, 13 wt.%, 14 wt.%,15 wt.%, 16 wt.%, 17 wt.%, 18 wt.%, 19 wt.%, 20 wt.%.

A commercially available acidic silica abrasive is Sylodent 783 available from W. R. Grace & Company, Baltimore, Md. Sylodent 783 has a pH of 3.4-4.2 when measured as a 5% by weight slurry in water. For use in the present invention, the silica material has an average particle size of less than 10 microns, e.g., 3-7 microns, e.g. about 5.5 microns.

### Water

Water is present in the oral compositions of the invention. Water, employed in the preparation of commercial oral compositions should be deionized and free of organic impurities. Water commonly makes up the balance of the compositions and includes 5% to 45%, e.g., 10% to 20%, e.g., 25 - 35%, by weight of the oral compositions. This amount of water includes the free water which is added plus that amount which is introduced with other materials such as with sorbitol or silica or any components of the invention. The Karl Fischer method is a one measure of calculating free water.

### Humectants

Within certain embodiments of the oral compositions, it is also desirable to incorporate a humectant to reduce evaporation and also contribute towards preservation by lowering water activity. Certain humectants can also impart desirable sweetness or flavor to the compositions. The humectant, on a pure humectant basis, generally includes 15% to 70% in one embodiment or 30% to 65% in another embodiment by weight of the composition.

Suitable humectants include edible polyhydric alcohols such as glycerine, sorbitol, xylitol, propylene glycol as well as other polyols and mixtures of these humectants. Mixtures of glycerine and sorbitol may be used in certain embodiments as the humectant component of the compositions herein.

### pH Adjusting Agents

In some embodiments, the compositions of the present disclosure contain a buffering agent. Examples of buffering agents include anhydrous carbonates such as sodium carbonate, sesquicarbonates, bicarbonates such as sodium bicarbonate, silicates, bisulfates, phosphates (e.g., monopotassium phosphate, monosodium phosphate, disodium phosphate, dipotassium phosphate, tribasic sodium phosphate, sodium tripolyphosphate, pentapotassium tripolyphosphate, phosphoric acid), citrates (e.g. citric acid, trisodium citrate dehydrate), pyrophosphates (sodium and potassium salts, e.g., tetrapotassium pyrophosphate) and combinations thereof. The amount of buffering agent is sufficient to provide a pH of about 5 to about 9, preferable about 6 to about 8, and more preferable about 7, when the composition is dissolved in water, a mouthrinse base, or a toothpaste base. Typical amounts of buffering agent are about 5% to about 35%, in one embodiment about 10% to about 30%, in another embodiment about 15% to about 25%, by weight of the total composition.

The present invention involves applying to the oral cavity a safe and effective amount of the compositions described herein.

The compositions and methods according to the invention (e.g., Composition 1.0 *et seq)* can be incorporated into oral compositions for the care of the mouth and teeth such as toothpastes, transparent pastes, gels, mouth rinses, sprays and chewing gum.

As used throughout, ranges are used as shorthand for describing each and every value that is within the range. Any value within the range can be selected as the terminus of the range. It is understood that when formulations are described, they may be described in terms of their ingredients, as is common in the art, notwithstanding that these ingredients may react with one another in the actual formulation as it is made, stored and used, and such products are intended to be covered by the formulations described.

The following examples further describe and demonstrate illustrative embodiments within the scope of the present invention. The examples are given solely for illustration and are not to be construed as limitations of this invention as many variations are possible without departing from the scope thereof.

### Example 1

### Biofilm Model

The active-attachment biofilm model has been previously described by Extercate et al. Caries Research 2010; 44: 372-379, The biofilm model consists of a metal lid with 24 clamps carrying hydroxyapatite (HAP) disks. The model is inoculated in 24-well plates with native saliva. Biofilms were formed via active recruitment of bacteria onto free-hanging HAP disks.

Treatment is performed after formation of a 24 h biofilm. Lactic acid production is determined to assess the residual metabolic activity of biofilms after repeated exposure to test products. The assay is conducted using a L-Lactate Assay Kit according to the manufacturer's protocol (Cayman Chemical Company, Cat. No. 700510).

Colony forming units (CFUs) are determined to assess the anti-bacterial efficacy of test solutions after repeated exposure to biofilms. HAP disks are removed from the lid and transferred to 1.5 ml CPW for sonication. CFUs are determined by colony counting. Statistical analysis was performed using Minitab 16 Software. ANOVA and Tukey test were performed on available CFU counts and lactic acid values.

### Example 2

Anti-bacterial efficacy of arginine alone and in combination with zinc in solutions and in toothpastes is tested by live counts using the biofilm model described in Example 1. The approximate number of live counts (CFUs) are as follows:

| **Toothpaste** | **CFU/ml** |
|---|---|
| 8% arginine, 1.0% zinc oxide and 0.5% zinc citrate* | 9.0 |
| 1.0% zinc oxide and 0.5% zinc citrate, and no arginine* | 9.25 |
| 8% arginine, and no zinc citrate or zinc oxide* | 9.60 |
| Placebo (no arginine, no zinc)* | 9.83 |
| 8% arginine, 1.0% zinc oxide and 0.5% zinc citrate, tauronal* | 9.16 |

| | |
|---|---|
| ^{∗} (not claimed) | |

The number of live counts in the (solution) groups which contain:

| **Solution** | **CFU/ml** |
|---|---|
| 4% arginine, 0.5% zinc oxide and 0.25% zinc citrate* | 6.84 |
| 0.5% zinc oxide and 0.25% zinc citrate, but which do not contain arginine* | 7.50 |
| 4% arginine, and no zinc citrate or zinc oxide* | 10.0 |
| 0.5% zinc oxide* | 9.65 |
| 0.25% zinc citrate* | 9.55 |
| Media* | 9.76 |

| | |
|---|---|
| ^{∗} (not claimed) | |

The above demonstrates the surprising effect which results from the complex of arginine, zinc oxide, and zinc citrate as defined in the claims.

While the above arginine/zinc complex groups show a lower number of live counts (e.g., CFUs) compared to the zinc groups (which did not include arginine), both groups demonstrate a decreased number of CFUs relative to toothpaste and solution groups which contain arginine (4% solution, 8% toothpaste, which did not include zinc), zinc oxide (0.5% solution, 1.0% toothpaste, which did not include arginine or zinc citrate) and zinc citrate (0.25% solution, 0.5% toothpaste, which did not include arginine or zinc oxide) and control.

Groups (toothpastes and solutions) with arginine only (not together with zinc) are not decreased in the number of live counts relative to control groups.

### Example 3

The anti-metabolic activity of arginine alone and combined with zinc on bacteria in solutions and in toothpastes is measured by lactic acid production during a 3-hour window after the last treatment. The bacterial metabolic activity, as measured by lactic acid production, decreases in (toothpaste) groups with:

| **Toothpaste** | **Lactate mM** |
|---|---|
| 8% arginine, 1.0% zinc oxide and 0.5% zinc citrate* | 1.75 |
| 1.0% zinc oxide and 0.5% zinc citrate, and no arginine* | 1.5 |
| 8% arginine, and no zinc citrate or zinc oxide* | 2.5 |
| Placebo (no arginine, no zinc)* | 2.5 |
| 8% arginine, 1.0% zinc oxide and 0.5% zinc citrate, tauronal* | 1.75 |

| | |
|---|---|
| ^{∗} (not claimed) | |

The decrease in the metabolic activity is relative to control, as well as toothpastes which employ arginine only (which did not include zinc), toothpastes which employ 0.5% zinc oxide only, and toothpastes which employ 0.25% zinc citrate only.

The bacterial metabolic activity, as measured by lactic acid production (solution) :

| **Solution** | **Lactate mM** |
|---|---|
| 4% arginine, 0.5% zinc oxide and 0.25% zinc citrate* | 1.0 |
| 0.5% zinc oxide and 0.25% zinc citrate, but which do not contain arginine* | 1.0 |
| 4% arginine, and no zinc citrate or zinc oxide* | 3.9 |
| 0.5% zinc oxide* | 6.25 |
| 0.25% zinc citrate* | 6.0 |
| Media* | 2.25 |

| | |
|---|---|
| ^{∗} (not claimed) | |

Groups (toothpastes and solutions) with arginine only (which did not include zinc) do not show a decrease in metabolic activity relative to control or placebo groups.

### Example 4

The antibacterial activity of serine and lysine is assessed individually and in combination with zinc and determined by lactic acid production during a 3-hour window after the last treatment according to Example 1.

| **Solutions** | **Log CFU/ml** |
|---|---|
| Negative control (water)* | 9.47 |
| 0.5% zinc oxide and 0.25% zinc citrate* | 6.80 |
| 0.5% zinc oxide and 0.25% zinc citrate, 4% serine* | 7.15 |
| 4% serine* | 9.45 |
| 0.5% zinc oxide and 0.25% zinc citrate, 4% lysine* | 6.23 |
| 4% lysine* | 9.37 |

| | |
|---|---|
| ^{∗} (not claimed) | |

The groups (solutions) with 4% serine (not together with zinc) and 4% lysine (not complexed with zinc), do not demonstrate a decrease in the number of CFUs relative to control solutions (water).

However, groups (solutions) containing 4% lysine, 0.5% zinc oxide and 0.25% zinc citrate, are decreased relative to control and variable groups.

Groups with: 4% serine, 0.5% zinc oxide and 0.25% zinc citrate, do not demonstrate a decrease in CFU counts relative to control or variable (solutions) groups.

### Example 5

The anti-metabolic activity of serine and lysine individually, and in combination with zinc, are tested. Using the biofilm model described in Example 1, antibacterial activity of serine and lysine is tested individually and in combination with zinc. The groups with 4% serine and 4% lysine alone (which did not include zinc) do not demonstrate a decrease in metabolic activity relative to control solutions.

| **Solutions** | **Lactate mM** |
|---|---|
| Negative control (water)* | 2.7 |
| 0.5% zinc oxide and 0.25% zinc citrate* | 0.70 |
| 0.5% zinc oxide and 0.25% zinc citrate, 4% serine* | 3.7 |
| 4% serine* | 2.4 |
| 0.5% zinc oxide and 0.25% zinc citrate, 4% lysine* | less than 0.5 |
| 4% lysine* | 2.7 |

| | |
|---|---|
| ^{∗} (not claimed) | |

Groups containing: 4% lysine, 0.5% zinc oxide and 0.25% zinc citrate, exhibit decreased metabolic activity relative to control groups (water) and variable groups.

Groups with 4% serine, complexed with 0.5% zinc oxide and 0.25% zinc citrate, do not exhibit a decrease in metabolic activity relative to control or variable groups

### Example 6

Tables 1 and 2 demonstrate that the amount of soluble zinc in solution is increased in the presence of arginine, serine, and lysine.

**Table 1: Solubility of zinc salts in presence of serine or lysine (not claimed).**

| | Soluble Zn (%) = R^{∗}M2/(10^{∗}M1) | | | | | |
|---|---|---|---|---|---|---|
| Sample | | M1 (mg) | M2 (g) | R (ppm) | Sol. Zn (abs.) | % sol. Zn (rel) |
| Water | | NA | NA | NA | NA | NA |
| 0.5% Zinc Oxide + 0.25% Zinc Citrate (pH 6.95) | | 99 | 14.54 0 | 7.800 | 0.114558 | 23.9% |
| 4% Serine + 0.5% zinc oxide + 0.25% zinc citrate (pH 6.67) | | NA | NA | NA | 0.479741 | 100% |
| 4% Serine (pH 5.8) | | NA | NA | NA | NA | NA |
| 4% Lysine + 0.5% Zinc Oxide + 0.25% Zinc Citrate (pH 6.99) | | NA | NA | NA | 0.479741 | 100% |
| 4% Lysine (pH 5.6) | | NA | NA | NA | NA | NA |

**Table 2: Solubility of zinc in presence of arginine, lysine or serine.**

| **Simple Solutions** | **Relative Soluble Zinc** |
|---|---|
| ZnO+ZnCit (pH 6.8) | 24% |
| ZnO+ZnCit (pH 10.6*) | 3% |
| Arg+ZnO+ZnCit (pH 10.7) | 19% |
| Lys+ZnO+ZnCit (pH 6.8) | 100% |
| Ser+ZnO+ZnCit (pH 6.6) | 100% |

### Example 7 (not claimed)

| **Description** | **Compound I** |
|---|---|
| Humectants | 20.0-25.0 |
| Nonionic Surfactant | 1.0-2.0 |
| Amphoteric Surfactant | 3.0-4.0 |
| Flavoring/Fragrance/Coloring Agent | 2.0-3.0 |
| Polymers | 10.0-15.0 |
| pH Adjusting Agents | 1.5-3.0 |
| Precipitated Calcium Carbonate | 35.0 |
| Zinc Citrate Trihydrate | 0.5 |
| Zinc Oxide | 1.0 |
| Sodium Fluoride - USP, EP | 0.32 |
| Arginine Bicarbonate | 13.86 |
| Demineralized Water | q.s. |

### Example 8 (not claimed)

### Dentifrice Formulations

| **Description** | **Compound A** | **Compound B** | **Compound C** | **Compound D** |
|---|---|---|---|---|
| Humectants | 25.0-40.0 | 25.0-40.0 | 25.0-40.0 | 25.0-40.0 |
| Anionic Surfactant | 1.0-3.0 | 1.0-3.0 | 1.0-3.0 | 1.0-3.0 |
| Flavoring/Fragrance/Coloring Agent | 2.5-4.0 | 2.5-4.0 | 2.5-4.0 | 2.5-4.0 |
| Polymers | 4.0-6.0 | 4.0-6.0 | 4.0-6.0 | 4.0-6.0 |
| pH Adjusting Agents | 5.0-6.0 | 5.0-6.0 | 5.0-6.0 | 5.0-6.0 |
| Synthetic Amorphous Precipitated Silica | 16.0 | 21.37 | 17.92 | 7.81 |
| Alumina | 0.02 | 0.01 | 0.01 | 0.01 |
| Silica | - | - | - | 15.0 |
| Lauryl Alcohol | 0.02 | 0.02 | 0.02 | 0.02 |
| Zinc Citrate | 0.5 | 0.5 | 0.5 | 0.5 |
| Zinc Oxide | 1.0 | 1.0 | 1.0 | 1.0 |
| Sodium Fluoride - USP, EP | 0.32 | 0.32 | 0.32 | 0.32 |
| L-Arginine | 5.0 | 5.0 | 5.0 | 5.0 |
| Demineralized Water | q.s. | q.s. | q.s. | q.s. |
| Total Amount | 100% | 100% | 100% | 100% |

| | | | | |
|---|---|---|---|---|
| Impurities in the Compounds above are present in less than 1.0 wt. %. | | | | |

### Example 9 (not claimed)

### Dentifrice Formulations

| **Description** | **Compound E** | **Compound F** | **Compound G** |
|---|---|---|---|
| Humectants | 25.0-40.0 | 25.0-40.0 | 25.0-40.0 |
| Anionic Surfactant | 1.0-3.0 | 1.0-3.0 | 1.0-3.0 |
| Nonionic Surfactant | 0.1-1.0 | 0.1-1.0 | 0.1-1.0 |
| Amphoteric Surfactant | 0.1-1.0 | 0.1-1.0 | 0.1-1.0 |
| Flavoring/Fragrance/Coloring Agent | 4.0-6.0 | 4.0-6.0 | 4.0-6.0 |
| Polymers | 0.1-2.0 | 0.1-2.0 | 0.1-2.0 |
| pH Adjusting Agents | 0.1-2.0 | 0.1-2.0 | 0.1-2.0 |
| Thickener | 6.0 | 6.5 | 7.0 |
| Alumina | 0.1 | 0.1 | 0.1 |
| Synthetic Amorphous Precipitate Silica | 17.6 | 8.8 | 22.4 |
| Silica | - | 15.0 | - |
| Benzyl Alcohol | 0.1 | 0.1 | 0.1 |
| Synthetic Amorphous Silica | 5.0 | 5.0 | 5.0 |
| Zinc Citrate | 0.5 | 0.5 | 0.5 |
| Zinc Oxide | 1.0 | 1.0 | 1.0 |
| Sodium Fluoride - USP, EP | 0.32 | 0.32 | 0.32 |
| L-Arginine | 1.5 | 1.5 | 1.5 |
| Demineralized Water | q.s. | q.s. | q.s. |
| Total Amount | 100% | 100% | 100% |

| | | | |
|---|---|---|---|
| Impurities in the Compounds above are present in less than 1.0 wt. %. | | | |

### Example 10 (not claimed)

| **Description** | **Compound H** |
|---|---|
| Humectants | 45.0-55.0 |
| Abrasives | 14.0-16.0 |
| Anionic Surfactant | 1.0-3.0 |
| Nonionic Surfactant | 0.1-1.0 |
| Amphoteric Surfactant | 1.0-2.0 |
| Flavoring/Fragrance/Coloring Agent | 1.0-3.0 |
| Polymers | 0.1-2.0 |
| pH Adjusting Agents | 0.1-2.0 |
| Silica Thickener | 5.0 |
| Benzyl Alcohol | 0.1 |
| Zinc Citrate Trihydrate | 0.5 |
| Zinc Oxide | 1.0 |
| Sodium Fluoride - USP, EP | 0.32 |
| L-Arginine | 1.5 |
| Demineralized Water | q.s. |

| | |
|---|---|
| ^{∗}Note the above "Demineralized water" represents the amount of free water (i.e., without calculating the amount of water associated with silica and/or other ingredients) | |

### Example 11 (not claimed)

| **Description** | **Compound I** |
|---|---|
| Humectants | 35.0-45.0 |
| Abrasives | 9.0-11.0 |
| Anionic Surfactant | 1.0-3.0 |
| Flavoring/Fragrance/Coloring Agent | 2.0-4.0 |
| Polymers | 3.0-8.0 |
| pH Adjusting Agents | 4.0-8.0 |
| Silica Thickener | 5.0-10.0 |
| Zinc Citrate Trihydrate | 0.5 |
| Zinc Oxide | 1.0 |
| Sodium Fluoride - USP, EP | 0.32 |
| L-Arginine | 5.0 |
| Demineralized Water | q.s. |

| | |
|---|---|
| ^{∗}Note the above "Demineralized water" represents the amount of free water (i.e., without calculating the amount of water associated with silica and/or other ingredients) | |

### Example 12 (not claimed)

| **Description** | **Compound J** | **Compound K** | **Compound L** |
|---|---|---|---|
| Humectants | 20.0-50.0 | 20.0-50.0 | 20.0-50.0 |
| Abrasives | 5.0-20.0 | 5.0-20.0 | 5.0-20.0 |
| Anionic Surfactant | 0.1-3.0 | 0.1-3.0 | 0.1-3.0 |
| Nonionic Surfactant | 0.1-1.0 | 0.1-1.0 | 0.1-1.0 |
| Amphoteric Surfactant | 0.1-2.0 | 0.1-2.0 | 0.1-2.0 |
| Flavoring/Fragrance/Coloring Agent | 0.1-5.0 | 0.1-5.0 | 0.1-5.0 |
| Polymers | 0.1-2.0 | 0.1-2.0 | 0.1-2.0 |
| pH Adjusting Agents | 0.1-2.0 | 0.1-2.0 | 0.1-2.0 |
| Thickener | 6.0 | 6.5 | 7.0 |
| Dental Type Silica | - | - | 15.0 |
| High Cleaning Silica | - | 15.0 | - |
| Synthetic Abrasive Silica | 10.0 | - | - |
| Synthetic Amorphous Silica | 5.0 | 5.0 | 5.0 |
| Benzyl Alcohol | 0.4 | 0.4 | 0.4 |
| Synthetic Amorphous Silica | 5.0 | 5.0 | 5.0 |
| Zinc Citrate Trihydrate | 0.5 | 0.5 | 0.5 |
| Zinc Oxide | 1.0 | 1.0 | 1.0 |
| Sodium Fluoride - USP, EP | 0.32 | 0.32 | 0.32 |
| L-Arginine | 1.5 | 1.5 | 1.5 |
| Demineralized Water | q.s. | q.s. | q.s. |

| | | | |
|---|---|---|---|
| ^{∗}Note the above "Demineralized water" represents the amount of free water (i.e., without calculating the amount of water associated with silica and/or other ingredients) | | | |

### Example 13 (not claimed)

In one representative formulation, a dentifrice comprises the following:
a. 1.0 wt. % zinc oxide
b. 0.5 wt. % zinc citrate
c. 1.5 wt.% or 5.0 wt. % L-arginine
d. about 1450ppm sodium fluoride; and
e. about 5 wt.% small particle silica (e.g., AC43)

Wherein the dentifrice is expected to reduce erosion and reduce hypersensitivity in the mouth.

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight. The amounts given are based on the active weight of the material.

## Claims

1. An oral care composition comprising:
a. about 4 wt% arginine, based on the total weight of the composition, the weight of the basic amino acid being calculated as free form,
b. zinc oxide and zinc citrate, wherein the weight ratio of the amount of zinc oxide to zinc citrate is 2:1,
c. a fluoride source, wherein the fluoride source is sodium fluoride or stannous fluoride from 0.1 wt% - 2 wt%, and
d. an abrasive silica.

2. The oral care composition of claim 1, wherein the arginine is in the L configuration.

3. The oral care composition of claim 1 or 2, wherein the fluoride source is a soluble fluoride salt which provides soluble fluoride in amount of 50 to 25,000 ppm fluoride, preferably wherein sodium fluoride provides soluble fluoride in an amount of about 1000ppm - 1500ppm.

4. The composition of any of claims 1-3, wherein the fluoride source is stannous fluoride.

5. The oral care composition of any of claims 1-4 further comprising a preservative selected from: benzyl alcohol, methylisothizolinone ("MIT"), sodium bicarbonate, sodium methyl cocoyl taurate (tauranol), lauryl alcohol,
and polyphosphate.

6. The oral care composition of claim 5 comprising benzyl alcohol in an amount of from 0.1- 0.8 wt %, based on the total weight of the composition.

7. The oral care composition of claim 6 comprising benzyl alcohol in an amount of from 0.3 - 0.5% wt %, based on the total weight of the composition.

8. The oral care composition of claim 7, wherein the benzyl alcohol is 0.4 wt% based on the total weight of the composition.

9. The oral care composition of any of claims 1-8, wherein the oral composition may be any of the following oral compositions selected from the group consisting of: a toothpaste or a dentifrice, a mouthwash or a mouth rinse, a topical oral gel, and a denture cleanser.

10. The oral care composition of any of claims 1-9 for use in a method to improve oral health comprising applying an effective amount of the oral composition to the oral cavity of a subject in need thereof, wherein the method is effective to:
i. reduce or inhibit formation of dental caries,
ii. reduce, repair or inhibit early enamel lesions, e.g., as detected by quantitative light- induced fluorescence (QLF) or electrical caries measurement (ECM),
iii. reduce or inhibit demineralization and promote remineralization of the teeth,
iv. reduce hypersensitivity of the teeth,
v. reduce or inhibit gingivitis,
vi. promote healing of sores or cuts in the mouth,
vii. reduce levels of acid producing bacteria,
viii. to increase relative levels of arginolytic bacteria,
ix. inhibit microbial bio film formation in the oral cavity,
x. raise and/or maintain plaque pH at levels of at least pH 5.5 following sugar challenge,
xi. reduce plaque accumulation,
xii. treat dry mouth,
xiii. enhance systemic health, including cardiovascular health,
xiv. Whiten teeth,
xv. reduce erosion of the teeth,
xvi. immunize (or protect) the teeth against cariogenic bacteria and their effects, and/or
xvii. clean the teeth and oral cavity.

## Patentansprüche

1. Eine Mundpflegezusammensetzung, umfassend:
a. 4 Gew.-% Arginin, bezogen auf das Gesamtgewicht der Zusammensetzung, wobei das Gewicht der basischen Aminosäure als freie Form berechnet wird,
b. Zinkoxid und Zinkcitrat, wobei das Gewichtsverhältnis der Menge an Zinkoxid zu Zinkcitrat 2:1 ist,
c. eine Fluoridquelle, wobei die Fluoridquelle Natriumfluorid oder Zinnfluorid ist, von 0,1 Gew.-% bis 2 Gew.-%, und
d. ein abrasives Siliziumdioxid.

2. Die Mundpflegezusammensetzung nach Anspruch 1, wobei das Arginin in der L-Konfiguration ist.

3. Die Mundpflegezusammensetzung nach Anspruch 1 oder 2, wobei die Fluoridquelle ein lösliches Fluoridsalz ist, das lösliches Fluorid in einer Menge von 50 bis 25.000 ppm Fluorid bereitstellt, vorzugsweise wobei Natriumfluorid lösliches Fluorid in einer Menge von etwa 1000ppm bis 1500ppm bereitstellt.

4. Die Zusammensetzung nach einem der Ansprüche 1-3, wobei die Fluoridquelle Zinnfluorid ist.

5. Die Mundpflegezusammensetzung nach einem der Ansprüche 1-4, ferner umfassend ein Konservierungsmittel, ausgewählt aus: Benzylalkohol, Methylisothiazolinon ("MIT"), Natriumbicarbonat, Natriummethylcocoyltaurat (Tauranol), Laurylalkohol und Polyphosphat.

6. Die Mundpflegezusammensetzung nach Anspruch 5, umfassend Benzylalkohol in einer Menge von 0,1 bis 0,8 Gew.-%, bezogen auf das Gesamtgewicht der Zusammensetzung.

7. Die Mundpflegezusammensetzung nach Anspruch 6, umfassend Benzylalkohol in einer Menge von 0,3 bis 0,5 Gew.-%, bezogen auf das Gesamtgewicht der Zusammensetzung.

8. Die Mundpflegezusammensetzung nach Anspruch 7, wobei der Benzylalkohol 0,4 Gew.-%, bezogen auf das Gesamtgewicht der Zusammensetzung, ist.

9. Die Mundpflegezusammensetzung nach einem der Ansprüche 1-8, wobei die orale Zusammensetzung eine der folgenden oralen Zusammensetzungen sein kann, ausgewählt aus der Gruppe bestehend aus: einer Zahnpasta oder einem Zahnputzmittel, einem Mundwasser oder einer Mundspülung, einem topischen oralen Gel, und einem Gebissreiniger.

10. Die Mundpflegezusammensetzung nach einem der Ansprüche 1-9 zur Verwendung in einem Verfahren um die Mundgesundheit zu verbessern, umfassend das Anwenden einer wirksamen Menge der oralen Zusammensetzung in der Mundhöhle einer Person, die diese benötigt, wobei das Verfahren wirksam ist, um:
i. Bildung von Zahnkaries zu reduzieren oder zu inhibieren,
ii.frühe Zahnschmelzläsionen, z. B. erfasst durch quantitative lichtinduzierte Fluoreszenz (QLF) oder elektrische Kariesmessung (ECM), zu reduzieren, zu reparieren oder zu inhibieren,
iii.Demineralisation zu reduzieren oder zu inhibieren und Remineralisation der Zähne zu fördern,
iv. Hypersensibilität der Zähne zu reduzieren,
v. Gingivitis zu reduzieren oder zu inhibieren,
vi. Heilung von Wunden oder Schnittwunden im Mund zu fördern,
vii. Niveaus säureproduzierender Bakterien zu reduzieren,
viii. relative Niveaus von arginolytischen Bakterien zu erhöhen,
ix. Bildung eines mikrobiellen Biofilms in der Mundhöhle zu inhibieren,
x.Plaque-pH-Niveaus auf einen pH von mindestens 5,5 nach Zuckerbelastung anzuheben und/oder zu halten,
xi. Plaque-Ansammlung zu reduzieren,
xii. Mundtrockenheit zu behandeln,
xiii.systemische Gesundheit, einschließlich der kardiovaskulären Gesundheit, zu steigern,
xiv. Zähne zu weißen,
xv. Erosion der Zähne zu reduzieren,
xvi.Zähne gegen kariogene Bakterien und deren Effekte zu immunisieren (oder zu schützen), und/oder
xvii. Zähne und Mundhöhle zu reinigen.

## Revendications

1. Composition de soins bucco-dentaires comprenant :
a. 4 % en poids d'arginine, par rapport au poids total de la composition, le poids de l'acide aminé basique étant calculé sous forme libre,
b. de l'oxyde de zinc et du citrate de zinc, le rapport pondéral de la quantité d'oxyde de zinc au citrate de zinc étant de 2:1,
c. une source de fluorure, la source de fluorure étant du fluorure de sodium ou du fluorure stanneux de 0,1 % en poids à 2 % en poids, et
d. une silice abrasive.

2. Composition de soins bucco-dentaires selon la revendication 1, dans laquelle l'arginine est dans la configuration L.

3. Composition de soins bucco-dentaires selon la revendication 1 ou 2, dans laquelle la source de fluorure est un sel de fluorure soluble qui fournit du fluorure soluble en une quantité de 50 à 25 000 ppm de fluorure, de préférence dans laquelle le fluorure de sodium fournit du fluorure soluble en une quantité d'environ 1 000 ppm à 1 500 ppm.

4. Composition selon l'une quelconque des revendications 1 à 3, dans laquelle la source de fluorure est le fluorure stanneux.

5. Composition de soins bucco-dentaires selon l'une quelconque des revendications 1 à 4 comprenant en outre un conservateur choisi parmi : l'alcool benzylique, la méthylisothizolinone (« MIT »), le bicarbonate de sodium, le methyl cocoyl taurate de sodium (tauranol), l'alcool laurylique et le polyphosphate.

6. Composition de soins bucco-dentaires selon la revendication 5, comprenant de l'alcool benzylique en une quantité de 0,1 à 0,8 % en poids, par rapport au poids total de la composition.

7. Composition de soins bucco-dentaires selon la revendication 6, comprenant de l'alcool benzylique en une quantité de 0,3 à 0,5 % en poids, par rapport au poids total de la composition.

8. Composition de soins bucco-dentaires selon la revendication 7, dans laquelle l'alcool benzylique représente 0,4 % en poids par rapport au poids total de la composition.

9. Composition de soins bucco-dentaires selon l'une quelconque des revendications 1 à 8, dans laquelle la composition buccale peut être l'une quelconque des compositions buccales suivantes choisies dans le groupe constitué par : une pâte dentifrice ou un dentifrice, un bain de bouche ou un rince bouche, un gel buccal topique et un nettoyant pour prothèses dentaires.

10. Composition de soins bucco-dentaires selon l'une quelconque des revendications 1 à 9 pour une utilisation dans un procédé pour améliorer la santé buccodentaire comprenant l'application d'une quantité efficace de la composition buccale à la cavité buccale d'un sujet en ayant besoin, dans laquelle le procédé est efficace pour :
i. réduire ou inhiber la formation de caries dentaires,
ii. réduire, réparer ou inhiber les lésions précoces de l'émail, par exemple telles que détectées par fluorescence induite par la lumière quantitative (QLF) ou mesure électrique des caries (ECM),
iii. réduire ou inhiber la déminéralisation et favoriser la reminéralisation des dents,
iv. réduire l'hypersensibilité des dents,
v. réduire ou inhiber la gingivite,
vi. favoriser la cicatrisation des plaies ou des coupures dans la bouche,
vii. réduire les niveaux de bactéries productrices d'acide,
viii. pour augmenter les niveaux relatifs de bactéries arginolytiques,
ix. inhiber la formation de biofilm microbien dans la cavité buccale,
x. augmenter et/ou maintenir le pH de la plaque à des niveaux d'au moins pH 5,5 après provocation au sucre,
xi. réduire l'accumulation de plaque,
xii. traiter la bouche sèche,
xiii. améliorer la santé systémique, y compris la santé cardiovasculaire,
xiv. blanchir les dents,
xv. réduire l'érosion des dents,
xvi. immuniser (ou protéger) les dents contre les bactéries cariogènes et leurs effets, et/ou
xvii. nettoyer les dents et la cavité buccale.
